(19) Europäisches Patentamt
European Patent Office
Office européen des brevets

(11) Veröffentlichungsnummer: **0 242 666**
**A1**

(12) # EUROPÄISCHE PATENTANMELDUNG

(21) Anmeldenummer: **87104956.5**

(22) Anmeldetag: **03.04.87**

(51) Int. Cl.⁴: **A01N 47/36**, C07D 285/12

(30) Priorität: **16.04.86 DE 3612830**

(43) Veröffentlichungstag der Anmeldung:
**28.10.87 Patentblatt 87/44**

(84) Benannte Vertragsstaaten:
**AT BE CH DE ES FR GB IT LI NL SE**

(71) Anmelder: **BASF Aktiengesellschaft**
**Carl-Bosch-Strasse 38**
**D-6700 Ludwigshafen(DE)**

(72) Erfinder: **Grossmann, Klaus, Dr.**
**Wilhelm-Busch-Strasse 5**
**D-6703 Limburgerhof(DE)**
Erfinder: **Jung, Johann, Prof.Dr.**
**Hardenburgstrasse 19**
**D-6703 Limburgerhof(DE)**
Erfinder: **Schulz, Guenter, Dr.**
**Carl-Bosch-Strasse 96**
**D-6700 Ludwigshafen(DE)**
Erfinder: **Sauter, Hubert, Dr.**
**Neckarpromenade 20**
**D-6800 Mannheim 1(DE)**
Erfinder: **Hofmeister, Peter, Dr.**
**Bernhard-Humblot-Strasse 12**
**D-6730 Neustadt(DE)**
Erfinder: **Tuerk, Wolfgang, Dr.**
**Wittelsbachstrasse 61**
**D-6700 Ludwigshafen(DE)**

(54) **Thiadiazolylharnstoff enthaltendes Mittel zur Entblätterung von Pflanzen.**

(57) Mittel zur Entblätterung von Pflanzen, gekennzeichnet durch einen Gehalt an einem 1,3,4-Thiadiazol-2-yl-harnstoff der Formel I

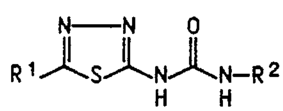

(I),

in der
$R^1$ für Wasserstoff oder Methyl und
$R^2$ für $C_1$-$C_6$-Alkyl, $C_3$-$C_8$-Cycloalkyl, $C_2$-$C_4$-Alkenyl, $C_3$-$C_6$-Alkinyl und Phenyl, das ggf. mit bis zu 2 Chlor- oder Fluoratomen, Methylgruppen, Trifluormethylgruppen oder Methoxygruppen substituiert ist, steht, Verfahren zur Herstellung des Mittels und seine Anwendung in einem Verfahren zur Entblätterung von Pflanzen.

EP 0 242 666 A1

Xerox Copy Centre

# Thiadiazolylharnstoff enthaltendes Mittel zur Entblätterung von Pflanzen

Die Erfindung betrifft Mittel zur Entblätterung von Pflanzen auf der Grundlage eines Thiadiazolylharnstoffs, Verfahren zur Herstellung dieser Mittel und ihre praktische Verwendung.

Für die Abtrennung von Pflanzenorganen wie Blättern, Blüten und Früchten vom Pflanzenkörper sind spezielle Gewebebereiche an der Basis der Blatt-bzw. Blüten-und Fruchtstiele verantwortlich. Bei Einsetzen des Trennungsprozesses werden die Wände der Zellen dieser Trennzone durch gesteigerte Pectinase-und Cellulasebildung weich, so daß eine Abtrennung des Organs (Abszission) durch mechanische Kräfte. z.B. durch Wind oder durch das eigene Gewicht möglich wird (Dörffling, Das Hormonsystem der Pflanzen, Georg Thieme Verlag, 1982).

1,2,3-Thiadiazol-5-yl-harnstoffe finden Verwendung als künstliches Abszissionsmittel (der Wirkstoff eines Handelsproduktes ist N-Phenyl-N'-(1,2,3-thiadiazol-5-yl)-harnstoff vgl. DE-OS 25 06 690 und 26 19 861). Der bisher verwendete Wirkstoff zeigt jedoch eine starke Abhängigkeit des Entblätterungserfolges von den Temperaturverhältnissen, was die Anwendung in Anbauzonen mit geringer Temperatur verhindert und durch die Unkalkulierbarkeit temporärer Temperaturschwankungen die Wirkungssicherheit stark beeinträchtigt. 1,3,4-Thiadiazol-2-yl-harnstoffe sind ebenfalls bekannt, z.B. aus J. Med. Chem. 22, 28ff; J. Med. Chem. 15, 1082 ff; DE-OS 19 23 939; JA-Pat. veröfft. 74-8253. Dort ist ihre Anwendung als Herbizide, Fungizide und pharmakologische Wirkstoffe beschrieben. Unbekannt ist ihre Verwendung als pflanzenverträgliche Abszissionsmittel zur gezielten Induktion des Abwurfes von Blättern, Blüten oder Früchten bei Kulturpflanzen, wie z.B. Baumwolle, Citrus, Oliven und Kern-und Steinobstsorten. Insbesondere bei Baumwolle besteht ein starkes wirtschaftliches Interesse an Abszissionsmitteln aus Gründen der Ernteerleichterung. Überraschenderweise übertreffen nun Mittel, die als Wirkstoff eine Verbindung der Formel I

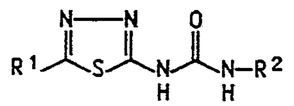 (I),

in der

$R^1$ für Wasserstoff oder Methyl und

$R^2$ für C1 - C6-Alkyl, $C_3$ -$C_8$-Cycloalkyl, $C_2$ -$C_4$-Alkenyl, $C_3$ -C6-Alkinyl und Phenyl, das ggf. mit bis zu 2 Chlor-oder Fluoratomen, Methylgruppen, Trifluormethylgruppen oder Methoxygruppen substituiert ist, steht, in wirksamer Konzentration enthalten, die bekannten Mittel, die auf isomeren Wirkstoffen basieren, erheblich in bezug auf Wirkungsintensität, Wirkungsgeschwindigkeit. Besonders ist die unerwartet deutliche Entblätterungswirkung bei relativ tiefen Temperaturen hervorzuheben, die die Anwendungssicherheit wesentlich erhöht.

Die Mittel werden den Pflanzen vornehmlich durch Blattspritzung zugeführt. Dabei kann die Ausbringung z.B. mit Wasser als Trägerstoff durch übliche Spritztechniken mit Spritzbrühenmengen von etwa 100 bis 1000 l/ha erfolgen. Eine Anwendung der Mittel im sogenannten "Low Volume"-und "Ultra-low-Volume"-Verfahren ist ebenso möglich wie ihre Applikation in Form von sogenannten Mikrogranulaten. Infolge der hohen Pflanzenverträglichkeit kann die Aufwandmenge an Wirkstoff stark variieren. Für die Blattbehandlung sind im allgemeinen Gaben von 0,01 bis 5 kg a. i./ha ausreichend.

Die Mittel können einfach durch Mischen einer wirksamen Menge einer Verbindung der Formel I mit geeigneten flüssigen oder festen Trägerstoffen und/oder Zusatz von oberflächenaktiven Stoffen erhalten werden.

Geeignete flüssige Trägerstoffe sind z.B. Wasser, aliphatische und aromatische Kohlenwasserstoffe, wie Benzol, Toluol, Xylol, Cyclohexanon, Isophoron, Dimethylsulfoxyd, Dimethylformiad, weiterhin Mineralölfraktionen. Als feste Trägerstoffe eignen sich Mineralerden, z.B. Tonsil, Silicagel, Talkum, Kaolin, Attaclay, Kalkstein, Kieselsäure und pflanzliche Produkte, z.B. Mehle.

An oberflächenaktiven Stoffen sind zu nennen: z.B. Calciumligninsulfonat, Polyoxyethylen-octylphenolether, Naphthalinsulfonsäuren und deren Salze, Phenolsulfonsäuren und deren Salze, Formaldehydkondensate, Fettalkoholsulfate sowie substituierte Benzolsulfonsäuren und deren Salze.

Der Anteil des bzw. der Wirkstoffe(s) in den konzentrierten Zubereitungen kann in weiten Grenzen variieren. Beispielsweise enthält ein Mittel etwa 10 bis 80 Gew.-% Wirkstoff, etwa 30 bis 90 Gew.-% flüssige oder feste Trägerstoffe sowie ggf. bis zu 20 Gew.-% oberflächenaktive Stoffe. Es kann bei Bedarf auf eine geeignete Anwendungskonzentration verdünnt werden.

Die zugrundeliegenden Wirkstoffe können auf bekannte Weise erhalten werden; z.B. durch die Umsetzung von 2-Amino-1,3,4-Thiadiazol oder 2-Amino-5-methyl-1,3,4-Thiadiazol mit einem geeigneten Isocyanat:

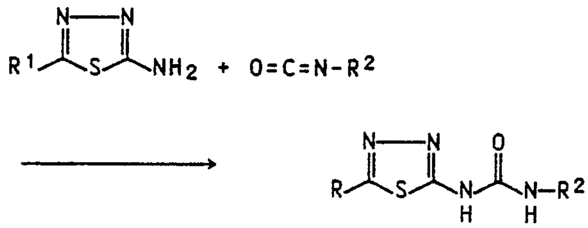

Dieser Reaktionstyp ist zusammenfassend z.B. beschrieben in "The chemistry of cyanates and their thio derivatives" Hrsg. S. Patai, (1977). Nachfolgend sind Beispiele für Verbindungen der Formel I in einer Liste zusammengestellt, der sich Beispiele zur biologischen Wirksamkeit der Mittel anschließen.

Die erfindungsgemäßen Mittel können entweder für sich allein, in Mischung mit anderen Mitteln oder mit anderen Wirkstoffen angewendet werden. Gegebenenfalls können andere Entblätterungs-, Pflanzenschutz-oder Schädlingsbekämpfungsmittel je nach dem gewünschten Zweck zugesetzt werden.

Tabelle 1: Verbindungen

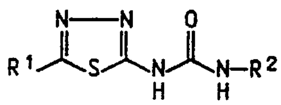

| Nr. | $R^1$ | $R^2$ | Fp. °C |
|---|---|---|---|
| 1 | H | $-CH_3$ | |
| 2 | H | $-C_4H_9$ | |
| 3 | H | $-CH(CH_3)_2$ | |
| 4 | H | $-CH_2CH(CH_3)_2$ | 171 |
| 5 | H | Cyclopentyl | 210-12 |
| 6 | H | Cyclohexyl | 211-14 |
| 7 | H | $-CH_2-CH=CH_2$ | 235-36 |
| 8 | H | $-C(CH_3)_2-C\equiv CH$ | 210 |
| 9 | H | $-C_6H_5$ | > 230 |
| 10 | H | $-(4-Cl-C_6H_4)$ | |
| 11 | H | $-(4-CH_3-C_6H_4)$ | |
| 12 | H | $-(2-F-C_6H_4)$ | > 230 |
| 13 | H | $-(3-Cl-C_6H_4)$ | > 230 |
| 14 | H | $-(3,4-Cl_2-C_6H_3)$ | > 230 |
| 15 | H | $-(3-CF_3-C_6H_4)$ | > 230 |
| 16 | H | $-(3-OCH_3-C_6H_4)$ | > 230 |
| 17 | $-CH_3$ | $-C_6H_5$ | > 230 |
| 18 | $-CH_3$ | $-(4-CH_3-C_6H_4)$ | |
| 19 | $-CH_3$ | $-(4-Cl-C_6H_4)$ | |
| 20 | $-CH_3$ | $-2(2-F-C_6H_4)$ | |

Anwendungsbeispiele

Beispiel A

Junge Baumwollpflanzen (Sorte Delta Pine, Entwicklungsstadium 5 -6 entwickelte Laubblätter) wurden unter Gewächshausbedingungen angezogen (Tag/Nachttemperatur 26/16°C, relat. Luftfeuchte 50 - 70%) und tropfnaß mit den unten angegebenen Wirkstoffen unter Zusatz von 1 % Citowett in wäßriger Lösung blattbehandelt. Fünf und sieben Tage nach Wirkstoffapplikation wurde die Anzahl abgeworfener Blätter und der Grad der Entblätterung in % zur Kontrolle angegeben.

| Mittel, enthaltend Wirkstoff Nr. | Umgerechnete Aufwandmenge kg/ha | % Entblätterung nach 5 | 7 Tagen |
|---|---|---|---|
| 9 formuliert in | 3 | 83 | 86 |
| 9 Tween 85 ⊕ Endmenge 5 Gew.-% | 5 | 95 | 98 |
| 17 formuliert in Tween 85 ⊕ Endmenge 5 Gew.-% | 5 | 82 | 84 |
| Vergleichsmittel | | | |
| N-Phenyl-N'-(1,2,3- | 3 | 36 | 45 |
| thiadiazol-5-yl)-harnstoff (Handelsprodukt) | 5 | 41 | 54 |
| Unbehandelt | - | 0 | 0 |

⊕ Tween 85 ist ein Netzmittel auf der Grundlage von Polyoxyethylensorbitan-Trioleat

Beispiel B

Junge Baumwollpflanzen wurden wie in Beispiel A unter Gewächshausbedingungen angezogen. Nach der Blattbehandlung - wie in Beispiel A beschrieben - mit den unten angegebenen Mitteln wurden die Pflanzen in speziellen Klimaräumen bei geringeren Temperaturbedingungen (Tag-/Nachttemperatur 22/13°C) weiterkultiviert. Eine Woche nach Applikation der Mittel wurde die Anzahl abgeworfener Blätter bestimmt und der Grad der Entblätterung in % zur Kontrolle angegeben.

| Mittel, enthaltend Wirkstoff Nr. | Umgerechnete Aufwandmenge kg/ha | % Entblätterung nach 1 Woche |
|---|---|---|
| 9 formuliert in Tween 85, ⊕ Endmenge 5 Gew.-% | 3 | 65 |
| Vergleichsmittel: N-Phenyl-N'-(1,2,3-thiadiazol-5-yl)-harnstoff (Handelsprodukt) | 3 | 9 |
| Unbehandelt | - | 0 |

Die Ergebnisse aus Beispiel A und B zeigen, daß die erfindungsgemäßen Mittel unter bestimmten Temperaturverhältnissen sowohl hinsichtlich Wirkungsgeschwindigkeit dem handelsüblichen Wirkstoff deutlich überlegen sind und ihre gute Wirkung als Entblätterungsmittel auch bei geringen Temperaturen entfalten.

## Ansprüche

1. Mittel zur Entblätterung von Pflanzen, gekennzeichnet durch einen Gehalt an einem 1,3,4-Thiadiazol-2-yl-harnstoff der Formel I

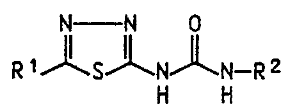

(I),

in der
$R^1$ für Wasserstoff oder Methyl und
$R^2$ for $C_1$ -$C_6$-Alkyl, $C_3$ -$C_8$-Cycloalkyl, $C_2$ -$C_4$-Alkenyl, $C_3$ -$C_6$-Alkinyl und Phenyl, das ggf. mit bis zu 2 Chlor- oder Fluoratomen, Methylgruppen, Trifluormethylgruppen oder Methoxygruppen substituiert ist, steht.

2. Verfahren zur Herstellung von Mitteln zur Entblätterung von Pflanzen, dadurch gekennzeichnet, daß man eine wirksame Menge der Verbindung der Formel I mit mindestens einem festen oder flüssigen Trägerstoff sowie ggf. mit einem oder mehreren oberflächenaktiven Mitteln mischt.

3. Verfahren zur Entblätterung von Pflanzen, dadurch gekennzeichnet, daß man ein Mittel gemäß Anspruch 1 auf Pflanzen oder deren Lebensraum einwirken läßt.

Europäisches Patentamt

# EUROPÄISCHER RECHERCHENBERICHT

Nummer der Anmeldung

EP 87 10 4956

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| X | FR-A-1 598 961 (BAYER)<br>* Patentansprüche; Seite 4, Zeilen 29-35 *<br>--- | 1-3 | A 01 N 47/36<br>C 07 D 285/12 |
| X | CH-A- 554 886 (AIR PRODUCTS)<br>* Spalte 5, Zeilen 2-3 *<br>--- | 1-3 | |
| X | FR-A-2 001 083 (MOBIL OIL)<br>* Patentanspruch 13 *<br>--- | 1,2 | |
| X | FR-A-2 003 832 (AIR PRODUCTS)<br>* Patentansprüche *<br>--- | 1,2 | |
| X | FR-A-2 007 069 (HOKKO CHEMICAL INDUSTRY)<br>* Patentansprüche *<br>--- | 1,2 | **RECHERCHIERTE SACHGEBIETE (Int. Cl.4)** |
| X | US-A-3 972 706 (W.R. ARNOLD)<br>* Patentanspruch 1; Spalte 16, Zeile 10 - Spalte 17, Zeile 51 *<br>--- | 1,2 | A 01 N |
| X | US-A-3 990 879 (Q.F. SOPER)<br>* Patentanspruch 1; Spalte 9, Zeilen 31-62 *<br>--- -/- | 1,2 | |

Der vorliegende Recherchenbericht wurde für alle Patentansprüche erstellt.

| Recherchenort | Abschlußdatum der Recherche | Prüfer |
|---|---|---|
| DEN HAAG | 13-08-1987 | DECORTE D. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

# EUROPÄISCHER RECHERCHENBERICHT

Nummer der Anmeldung

EP 87 10 4956

Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | CENTRAL PATENTS INDEX, BASIC ABSTRACTS JOURNAL, Sektion C, Woche V12, 1974, Zusammenfassung Nr. 22089V/12, Derwent Publications Ltd, London, GB; & JP-A-74 008 253 (KUMIAI CHEMICAL IND CO) 25-02-1974 (Kat. D) | 1,2 | |
| | ----- | | |
| | | | RECHERCHIERTE SACHGEBIETE (Int. Cl.4) |

Der vorliegende Recherchenbericht wurde für alle Patentansprüche erstellt.

| Recherchenort | Abschlußdatum der Recherche | Prufer |
|---|---|---|
| DEN HAAG | 13-08-1987 | DECORTE D. |

KATEGORIE DER GENANNTEN DOKUMENTE

- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument

EPA Form 1503 03 82